# EUROPÄISCHE PATENTSCHRIFT

(11) **EP 1 979 733 B1**
(45) Veröffentlichungstag und Bekanntmachung des Hinweises auf die Patenterteilung: **27.06.2018**
(21) Anmeldenummer: 07703093.0
(22) Anmeldetag: 29.01.2007
(51) Int. Cl.: G01N 15/06, G01N 33/28, F16N 29/04

(54) **VORRICHTUNG ZUM DETEKTIEREN VON PARTIKELN IN EINEM FLUIDSTROM SOWIE ZUGEHÖRIGES SYSTEM ZUM KÜHLEN UND/ODER SCHMIEREN**
APPARATUS FOR DETECTING PARTICLES IN A FLUID FLOW AND ASSOCIATED COOLING AND/OR LUBRICATING SYSTEM
DISPOSITIF DE DÉTECTION DE PARTICULES DANS UN COURANT DE FLUIDE ET SYSTÈME CORRESPONDANT POUR REFROIDIR ET/OU LUBRIFIER

(30) Priorität: 02.02.2006 DE 102006005956
(43) Veröffentlichungstag der Anmeldung: 15.10.2008
(73) Patentinhaber: HYDAC FILTERTECHNIK GMBH, 66280 Sulzbach/Saar (DE)
(72) Erfinder: MANNEBACH, Horst, 56294 Münstermaifeld (DE); JIRGAL, Mathias, Leo, 66126 Saarbrücken (DE); KLEBER, Jörg, 66538 Neunkirchen (DE)
(74) Vertreter: Crazzolara, Helmut
(86) Internationale Anmeldenummer: PCT/EP2007/000730
(87) Internationale Veröffentlichungsnummer: WO 2007/088015

(56) Entgegenhaltungen:
- EP-A1- 0 773 440
- EP-A2- 0 091 034
- DD-A1- 204 317
- GB-A- 2 101 330
- US-A1- 5 001 424

## Beschreibung

Die Erfindung betrifft eine Vorrichtung zum Detektieren von Partikeln in einem Fluidstrom, insbesondere einen induktiven Partikelzähler, gemäß dem Oberbegriff des Anspruchs 1, sowie ein zugehöriges System zum Kühlen und/oder Schmieren von Komponenten einer Antriebseinheit mittels eines Fluids gemäß dem nebengeordneten Anspruch.

Gattungsgemäße Vorrichtungen sind beispielsweise aus der US 5 001 424 bekannt. Die bekannte Vorrichtung weist zwei Feldspulen auf, welche -- bezogen auf den Fluidstrom -- axial hintereinander angeordnet sind. Axial in der Mitte zwischen den beiden Feldspulen ist eine Sensorspule angeordnet. Die Sensorspule hat den gleichen radialen Abstand zu der das Fluid führenden Leitung wie die Feldspulen. Infolge der Erregung eines magnetischen Feldes durch die Feldspule können mit der Sensorspule sowohl ferromagnetische als auch nicht ferromagnetische, elektrisch leitfähige Partikel in dem Fluidstrom detektiert werden können.

Die erfindungsgemäße Vorrichtung ist vorzugsweise einsetzbar in einem System zum Kühlen und/oder Schmieren von Komponenten einer Antriebseinheit mittels eines Fluids, insbesondere zum Kühlen und/oder Schmieren eines Getriebes einer Windkraftanlage. Die Wartung derartiger Systeme ist aufgrund des hoch gelegenen Einbauortes der erfindungsgemäßen Vorrichtung besonders aufwändig. Es ist daher wünschenswert, zum einen den Wechsel des Kühl- und/oder Schmiermittels, beispielsweise eines Kühl- und/oder Schmieröles, nur dann vorzunehmen, wenn dies aufgrund der durch Abrieb der mechanischen Komponenten der Antriebseinheit oder durch Schmutzeintrag von außen hervorgerufenen Partikelbelastung zweckmäßig oder erforderlich ist. Die erfindungsgemäße Vorrichtung muss daher ein dauerhaft zuverlässiges Detektieren der Partikel gewährleisten. Hierzu gehört auch die Möglichkeit, verhältnismäßig kleine Partikel zuverlässig zu detektieren.

Die EP 0 091 034 A2 zeigt eine Anordnung zur Erfassung von Metallgegenständen in einem Materialfluss unter Verwendung einer Suchspule. Auf dem Umfang eines Rohres, durch das der Materialfluss erfolgt, sind die Spulen der Suchspule platziert und zwar eine Primärspule in der Mitte und in Achsrichtung des Rohres davor und dahinter im gleichen Abstand von der Primärspule je eine Sekundärspule. Die Primärspule wird von einem Wechselstrom gespeist. Durch den symmetrischen Aufbau der Suchspule und die Gegeneinanderschaltung der Sekundärspule wird erreicht, dass das Ausgangssignal der Suchspule ungefähr Null ist, solange sich im Erfassungsbereich der Spule keine magnetischen oder elektrisch leitenden Gegenstände befinden. Wenn ein Metallpartikel den Erfassungsbereich passiert, entsteht ein Ausgangssignal in Form einer Differenzspannung.

Die DD-WP 204 317 zeigt eine Anordnung zur Metallsuche in Stoffströmen, die auf dem Prinzip der Induktion im wechselnden Magnetfeld beruht und aus vier Spulen besteht, wobei zwei Spulen Generatorspulen und zwei weitere Spulen so genannte Suchspulen sind. Die Generator- und Suchspulen werden so zueinander angeordnet, dass ein durch die Generatorspulen fließender Wechselstrom ein wechselndes Magnetfeld erzeugt, in dessen Kraftlinienverlauf die Suchspulen angeordnet sind. Dabei werden die zwei Suchspulen von den Kraftlinien der Generatorspulen in entgegengesetzter Richtung durchlaufen. Eine in den Suchspulen induzierte Spannung bzw. deren zeitlicher Mittelwert wird mittels eines Komparators mit einem Vergleichswert verglichen und bei Überschreiten eines eingestellten Schwellwertes wird ein Schaltvorgang ausgelöst.

Durch die US 2003/221911 A1 wird ein Überwachungssystem für Schmiermittel aufgezeigt, bei dem der Zustand eines flüssigen Schmiermittels in einer Schmiermittelkammer einer Radnabe überwacht wird. Dabei werden zwei jeweils auf einer Scheibe aufgebrachte planare Spiralspulen so gegeneinander beabstandet angeordnet, dass der entstehende Spalt zwischen den Spulen eine induktive Kopplung bewirkt, wenn der Spalt mit einem dielektrischen Material, beispielsweise Öl, gefüllt ist. Eine der beiden Spulen wird bestromt und in der gegenüberliegenden Spule wird eine Spannung induziert. Partikel innerhalb des Kanals zwischen den beiden Spulen bewirken eine Änderung der induzierten Spannung. Dieser Effekt ermöglicht, Partikel im Schmiermittel zu detektieren.

Die DE 39 31 497 A1 zeigt eine Vorrichtung zum Erfassen von Verschmutzungen in Fluiden, insbesondere Schmierstoffen, auf. Innerhalb eines von dem Fluid durchflossenen Spulenkörpers wird mittels eines Drallkörpers eine derartige Strömung erzeugt, dass in dem Fluid befindliche Partikel sich bevorzugt in der Nähe des Innenrandes des Spulenkörpers bewegen. Dergestalt wird eine hohe Empfindlichkeit des verwendeten Spulensystems in diesen Bereich ausgenutzt. Durch elektronische Auswertung der von dem induktiv arbeitenden Sensor abgegebenen Signale ist es möglich, die Größe der unerwünschten Verunreinigungen zu bestimmen.

Die EP 0 103 655 A1 zeigt eine Anordnung zur Bestimmung der Eigenschaften magnetischer Partikeldispersionen auf.

Der Erfindung liegt daher die Aufgabe zugrunde, eine Vorrichtung und ein zugehöriges System bereitzustellen, welche die Nachteile des Standes der Technik überwinden. Insbesondere soll die Partikelbelastung des Fluids dauerhaft zuverlässig detektierbar sein. In einem Ausführungsbeispiel sollen auch verhältnismäßig kleine Partikel mit einer Größe von weniger als 100 *µ*m dauerhaft zuverlässig detektiert werden können.

Diese Aufgabe ist durch die im Anspruch 1 bestimmte Vorrichtung sowie durch das im nebengeordneten Anspruch bestimmte System gelöst. Besondere Ausführungsarten der Erfindung sind in den Unteransprüchen bestimmt.

Gemäß dem Kennzeichen von Anspruch 1 ist vorgesehen, dass der in Bezug der in Bezug auf die Strömungsrichtung und in Bezug auf die Mittelachse der Leitung radiale Abstand der Sensorspulen von dem Fluidstrom von dem radialen Abstand der Feldspule von dem Fluidstrom abweicht.

Der radiale Abstand der Sensorspulen ist abweichend vom radialen Abstand der Feldspule, jeweils bezogen auf die Strömungsrichtung des Fluids. Zur Erhöhung der Empfindlichkeit können die Sensorspulen insbesondere einen geringeren radialen Abstand von dem Fluidstrom als die Feldspule aufweisen. Hierzu können die Sensorspulen beispielsweise auf einen ersten Wickelkörper gewickelt sein, der einen Abschnitt einer Leitung umgibt, innerhalb der das Fluid strömt.

Die Sensorspulen sind koaxial zu der Feldspule angeordnet. Die Sensorspulen sind dadurch symmetrisch zu dem von der Feldspule erzeugten Magnetfeld angeordnet.

Neben der mindestens einen Feldspule zum Erzeugen eines den Fluidstrom mindestens abschnittsweise abdeckenden Magnetfeldes weist die Vorrichtung mindestens zwei Sensorspulen auf, die gegensinnig gewickelt sind. Dadurch wird die Empfindlichkeit hinsichtlich der zu detektierenden Partikel erhöht und es können beispielsweise auch kleinere Partikel mit einer Größe von 50 bis 100 *µ*m detektiert werden. Eine erste Anschlussleitung der zweiten Sensorspule ist mit einer zweiten Anschlussleitung der ersten Sensorspule verbunden. In einem Ausführungsbeispiel sind die beiden Sensorspulen einstückig ausgebildet, d.h. mittels eines einzigen Drahtstückes gewickelt. Der Signalabgriff erfolgt zwischen der ersten Anschlussleitung der ersten Sensorspule und der zweiten Anschlussleitung der zweiten Sensorspule. In den beiden Sensorspulen wird infolge der Erzeugung eines Magnetfeldes durch die Feldspule eine Sensorspannung induziert, deren zeitlicher Verlauf abhängig ist von einem in dem Fluidstrom transportierten Partikel.

Die gemessene Partikelbelastung kann auch dahingehend ausgewertet werden, dass daraus Rückschlüsse gezogen werden, in welchem Zustand sich einzelne Komponenten des Systems befinden. So kann beispielsweise eine erhöhte Partikelbelastung ein Indiz dafür sein, dass eine oder mehrere Komponenten einen erhöhten Verschleiß aufweisen. Für diese Auswertung kann es zweckdienlich sein, zwischen magnetischen und elektrisch leitfähigen Partikel unterscheiden zu können, um dadurch die betroffene Komponente zu ermitteln. Auf diese Weise können sich anbahnende Schäden frühzeitig erkannt werden und unerwartete Betriebsausfälle beispielsweise durch rechtzeitigen Austausch der verschlissenen Komponenten vermieden werden.

Aufgrund der gegensinnigen Wicklung der beiden Sensorspulen ist es möglich, auch mit nur einer Feldspule Partikel zu detektieren. Anhand der Sensorspannung, insbesondere des zeitlichen Verlaufes der Sensorspannung, kann außerdem unterschieden werden, ob sich der Partikel im Abschnitt der ersten Sensorspule und/oder im Abschnitt der zweiten Sensorspule befindet. Dies ist besonders vorteilhaft, wenn die beiden Sensorspulen in Bezug auf eine Strömungsrichtung des Fluids mit einem axialen Versatz zueinander angeordnet sind, insbesondere axial voneinander beabstandet sind. In diesem Fall kann durch Auswertung des Sensorspulensignals auch die Strömungsgeschwindigkeit des Partikels und dadurch der Volumenstrom des Fluids ermittelt werden. Grundsätzlich können die beiden Sensorspulen hierzu beliebig verschaltet werden. In einem Ausführungsbeispiel sind die beiden Sensorspulen in Reihe geschaltet.

In einem Ausführungsbeispiel liegt der erste Wickelkörper nicht unmittelbar an der Leitung an, sondern ist von der Leitung mechanisch entkoppelt. Hierzu können vorzugsweise elastisch verformbare Abstandhalter zwischen dem ersten Wickelkörper und der Leitung vorgesehen sein, die lediglich in punkt- und/oder linienförmiger Anlage an dem ersten Wickelkörper und/ oder an der Leitung sein können. In einem Ausführungsbeispiel kann der erste Wickelkörper beispielsweise durch mindestens einen, vorzugsweise mehrere, auf die Leitung aufgeschobenen oder aufgesteckten O-Ring beabstandet sein, der entweder an der insbesondere hohlzylindrischen Innenfläche des ersten Wickelkörpers anliegt oder zum Zweck der axialen Positionierung und Fixierung teilweise in eine umlaufende Nut auf der Innenseite des ersten Wickelkörpers einsetzbar ist. Alternativ oder ergänzend können auf der der Leitung zugewandten Innenseite des ersten Wickelkörpers auch vorzugsweise einstückig von dem ersten Wickelkörper ausgebildete Abstandhalter vorgesehen sein. Durch die mechanische Entkopplung wird eine Deformation des ersten Wickelkörpers und damit der Sensorspulen infolge von Druckschwankungen oder Druckpulsationen im Fluid verhindert, die andernfalls zu einem Störsignal in den Sensorspulen führen können. Beispielsweise könnten langsame Druckschwankungen oder auch statische Druckänderungen das Offsetsignal der Sensorspulen verschieben, oder Druckpulsationen könnten als Partikel interpretiert werden. Vorzugsweise ist an der axialen Position der Abstandshalter radial außen eine vorzugsweise umlaufende Abstützung für den ersten Wickelkörper vorgesehen, beispielsweise ein Armierungselement. Dadurch können die im Bereich des Abstandshalters eingeleiteten Kräfte aufgenommen werden und eine das Sensorsignal verfälschende Wirkung von Druckschwankungen oder Druckpulsationen vermieden werden.

In einem Ausführungsbeispiel sind die Feldspule und die Sensorspulen in Bezug auf die Strömungsrichtung des Fluids axial zueinander verschiebbar. Insbesondere kann hierfür eine Einstelleinrichtung vorgesehen sein, mittels der beispielsweise die Feldspule in Bezug auf die beiden Sensorspulen axial verschiebbar ist. Die Feldspule kann auf einen zweiten Wickelkörper gewickelt sein, der mittels der Einstelleinrichtung gegenüber dem ersten Wickelkörper verschiebbar ist. Hierzu kann beispielsweise ein Einstellelement auf den ersten Wickelkörper aufschraubbar sein, und das Einstellelement kann form- oder kraftschlüssig mit dem zweiten Wickelkörper verbunden sein. Dadurch ist bei einem Drehen des Einstellelements auf dem ersten Wickelkörper der zweite Wickelkörper in seiner Axialposition verschiebbar. Durch die Einstellmöglichkeit der axialen Position der Feldspule in Bezug auf die Sensorspulen kann das Offsetsignal der Sensorspulen eingestellt werden, insbesondere zu Null gemacht werden.

In einem Ausführungsbeispiel weist die Vorrichtung zwei Feldspulen auf. Die beiden Feldspulen sind miteinander verbunden, insbesondere einstückig aus einem einzigen Stück Draht gewickelt. Grundsätzlich können die beiden Feldspulen auf verschiedene Weise miteinander verschaltet sein, insbesondere auch parallelgeschaltet sein. In einem Ausführungsbeispiel sind die beiden Feldspulen in Reihe geschaltet und werden von einem gemeinsamen Feldspulenstrom erregt. In einem Ausführungsbeispiel sind die beiden Feldspulen außerdem gleichsinnig gewickelt, was insbesondere in Verbindung mit den beiden gegensinnig gewickelten Sensorspulen besonders vorteilhaft für das Detektieren der Partikel ist.

Anstelle von zwei Sensorspulen und/oder ein oder zwei Feldspulen können auch mehrere Sensorspulen und/oder mehrere Feldspulen vorgesehen sein. Vorzugsweise weisen mindestens zwei Sensorspulen, die gegensinnig gewickelt sind, die gleiche Induktivität auf, insbesondere denselben Durchmesser, dieselbe Wicklungsdichte (Anzahl der Wicklungen pro Länge) und dieselbe Wicklungsdrahtstärke, wodurch sich im Idealfall ein verschwindendes Offsetsignal ergibt. Es können sowohl Partikel aus einem magnetischen Werkstoff detektiert werden, beispielsweise aus einem ferro- oder ferrimagnetischen Werkstoff, als auch Partikel aus einem nicht-magnetischen Werkstoff, sofern dieser elektrisch leitfähig ist.

Von der Verbindung der beiden Sensorspulen, gegebenenfalls auch von der Verbindung der beiden Feldspulen, kann zu Detektionszwecken und vor allem zu Prüfzwecken eine Anschlussleitung nach außen geführt werden. Dadurch können die so einzeln kontaktierbaren Spulen beispielsweise auf Durchgang und/oder Kurzschluss geprüft werden.

Die Erfindung betrifft auch ein System zum Kühlen und/oder Schmieren von Komponenten einer Antriebseinheit mittels eines Fluids, insbesondere zum Kühlen und/oder Schmieren eines Getriebes einer Windkraftanlage. Zu einem solchen System gehören neben einer Fluidpumpe und einem Fluidfilter auch die zu kühlende und/oder zu schmierende Einheit, beispielsweise ein Lager oder ein Zahnradpaar. Das Kühl- und/oder Schmiermittel wird in der Regel in einem Kreislauf gepumpt, in dem auch die erfindungsgemäße Vorrichtung zum Detektieren der Partikel angeordnet ist.

In einem Ausführungsbeispiel ist die Detektionsvorrichtung zwischen der Fluidpumpe und dem Fluidfilter angeordnet. Besonders vorteilhaft ist, wenn die Detektionsvorrichtung im Bereich eines Verbindungselements, insbesondere eines Verbindungsflansches zwischen Pumpe und Filter angeordnet ist, weil dadurch eine Nachrüstung bekannter Systeme mit einer erfindungsgemäßen Detektionsvorrichtung einfach möglich ist. Pumpe, Verbindungselement und Filter bilden darüber hinaus in der Regel eine Bau- und Liefereinheit, wohingegen das zu kühlende und/oder zu schmierende Getriebe den Auffangraum, beispielsweise einen Getriebesumpf oder eine Ölwanne, und die Fluidführung umfasst. In diesem Fall kann die erfindungsgemäße Baueinheit aus Pumpe, Verbindungselement mit Detektionsvorrichtung und Filter von außen an das Getriebe angebaut werden.

Alternativ oder ergänzend kann die erfindungsgemäße Detektionsvorrichtung auch zwischen dem Fluidfilter und einer zu kühlenden und/oder zu schmierenden Einheit angeordnet sein und/oder zwischen der zu kühlenden und/oder zu schmierenden Einheit und einer Rücklaufleitung zur Fluidpumpe.

Weitere Vorteile, Merkmale und Einzelheiten der Erfindung ergeben sich aus den Unteransprüchen und der nachfolgenden Beschreibung, in der unter Bezugnahme auf die Zeichnungen mehrere Ausführungsbeispiele im Einzelnen beschrieben sind. Dabei können die in den Ansprüchen und in der Beschreibung erwähnten Merkmale jeweils einzeln für sich oder in beliebiger Kombination erfindungswesentlich sein.
- Fig. 1: zeigt einen Längsschnitt durch ein erstes Ausführungsbeispiel einer erfindungsgemäßen Vorrichtung;
- Fig. 2: zeigt schematisch die Anordnung der Sensorspulen in Bezug auf die Feldspulen der Fig. 1;
- Fig. 3: zeigt einen Längsschnitt durch ein zweites Ausführungsbeispiel einer erfindungsgemäßen Vorrichtung;
- Fig. 4: zeigt schematisch die Anordnung der Sensorspulen in Bezug auf die Feldspule der Fig. 3; und
- Fig. 5: zeigt ein erfindungsgemäßes System zum Kühlen und/oder Schmieren von Komponenten einer Antriebseinheit mittels eines Fluids.

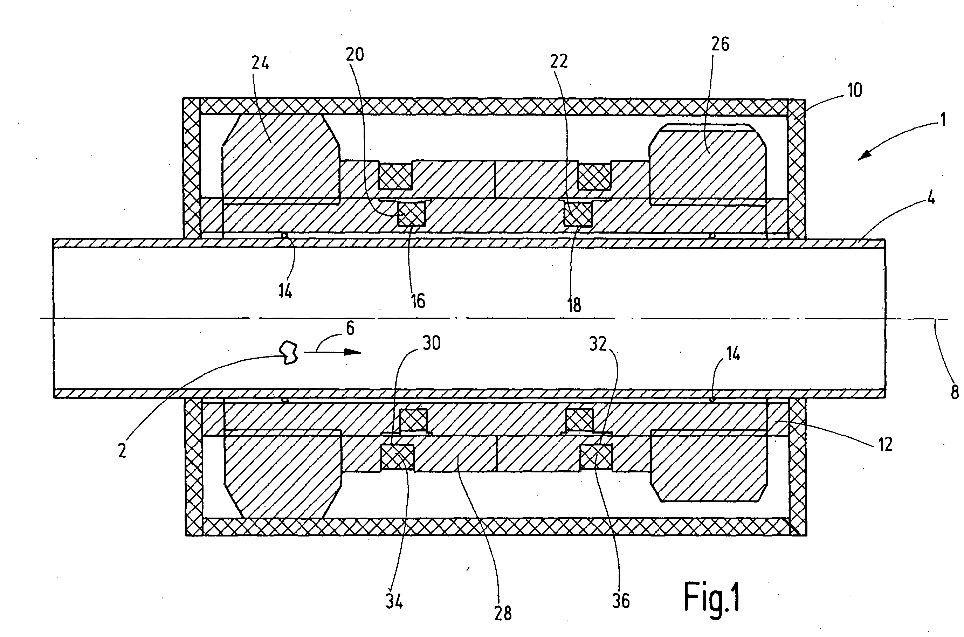

Die Fig. 1 zeigt einen Längsschnitt durch ein erstes Ausführungsbeispiel einer erfindungsgemäßen Vorrichtung 1 zum Detektieren von Partikeln 2 in einem in einer Leitung 4 strömenden Fluidstrom 6. Die Leitung 4 ist geradlinig mit einer Längsachse 8 und vorzugsweise aus einem nicht magnetischen und elektrisch nicht leitfähigen Werkstoff wie beispielsweise Keramik oder Kunststoff. Die Vorrichtung 1 ist außenseitig an der Leitung 4 angeordnet und festgelegt mit einem Gehäuse 10, dessen Werkstoff vorzugsweise so gewählt ist, dass sich eine gute Abschirmung der Vorrichtung 1 ergibt, beispielsweise aus einem Werkstoff mit hoher Anfangspermeabilität und geringer Leitfähigkeit wie etwa Ferrit.

Ein erster Wickelkörper 12 ist in geringem radialen Abstand zu der Leitung 4 angeordnet und mit seinen beiden Stirnenden in Anlage an dem Gehäuse 10 und dadurch axial fixiert. Der radiale Abstand zwischen dem ersten Wickelkörper 12 und der Leitung 4 ist durch zwei um die Leitung 4 umlaufende Abstandshalterelemente 14 bestimmt, die sowohl in Anlage an der Außenfläche der Leitung 4 als auch in Anlage an der Innenfläche des ersten Wickelkörpers 12 sind. Die Abstandshalterelemente 14 können beispielsweise jeweils durch einen O-Ring gebildet sein aus einem elastisch, insbesondere weichelastisch verformbaren Kunststoffwerkstoff. Der radiale Abstand sollte dabei so klein wie möglich gewählt werden, um eine hohe Empfindlichkeit der Vorrichtung 1 zu gewährleisten, und andererseits so groß wie nötig, um eine mechanische Entkopplung des ersten Wickelkörpers 12 von der Leitung 4 zu gewährleisten. Abweichend von der dargestellten Anordnung des Abstandshalterelements 14 ist es auch möglich, an der für das Abstandshalterelement 14 vorgesehenen axialen Position einen radialen Vorsprung und/oder eine radiale Vertiefung an der Leitung und/ oder an dem ersten Wickelkörper 12 vorzusehen, insbesondere eine umlaufende Nut an dem Wickelkörper 12 vorzusehen, in welche das Abstandshalterelement 14 teilweise eingelegt werden kann.

Auf der Außenseite weist der erste Wickelkörper 12 zwei Wickelfenster 16, 18 auf, die als im Längsschnitt rechteckförmige umlaufende Nuten eingeformt sind und die von der axialen Mitte des ersten Wickelkörpers 12, die auch die axiale Mitte der Vorrichtung 1 bildet, gleich beabstandet sind. In das erste Wickelfenster 16 ist die erste Sensorspule 20 eingelegt und in das zweite Wickelfenster 18 die zweite Sensorspule 22. Die beiden Sensorspulen 20, 22 sind aus demselben Spulendraht gewickelt, insbesondere aus einem einzigen Stück Spulendraht gewickelt. Die Sensorspulen 20, 22 weisen dieselbe Wicklungsdichte (Anzahl der Wicklung pro Längeneinheit) auf und besitzen dieselbe Induktivität. Die Sensorspulen 20, 22 liegen radial vertieft in dem ersten Wickelkörper 12 und sind dadurch mechanisch geschützt angeordnet. Die nach außen führenden Anschlussleitungen der beiden Sensorspulen 20, 22 sind nicht dargestellt.

Auf der Außenseite des zylindrischen, insbesondere kreiszylindrischen ersten Wickelkörpers 12 ist ein vorzugsweise durchgehendes Außengewinde angebracht. Axial endseitig ist jeweils ein erstes und gegenüberliegend ein zweites Einstellelement 24, 26 aufgeschraubt, das im Ausführungsbeispiel von jeweils einer Sechskantmutter gebildet ist. Die Einstellelemente 24, 26 sind dabei axial so positioniert, dass sie im Bereich der Abstandshalterelemente 14 liegen, um zu verhindern, dass die dort im Fall von Druckschwankungen oder Druckpulsationen auftretenden insbesondere radialen Kräfte zu einer Verfälschung des Ausgangssignals der Vorrichtung 1 führen. Etwa mittig in Bezug auf die axiale Länge des ersten Wickelkörpers 12 ist auf diesen ein zweiter Wickelkörper 28 aufgeschoben, der mittels der beiden Einstellelemente 24, 26, die gemeinsam die Einstelleinrichtung der Vorrichtung 1 bilden, in Richtung der Längsachse 8 verschiebbar ist.

Der zweite Wickelkörper 28 kann - wie im Ausführungsbeispiel dargestellt - zweiteilig ausgebildet sein; alternativ hierzu ist auch eine einstückige Ausbildung des zweiten Wickelkörpers 28 möglich. Mit seiner hohlzylindrischen Innenseite liegt der zweite Wickelkörper 28 an der Außenfläche des ersten Wickelkörpers 12 an. Auf seiner Außenseite weist der zweite Wickelkörper 28 zwei Wickelfenster 30, 32 auf, die als im Längsschnitt rechteckförmige umlaufende Nuten ausgebildet sind und in welche die erste Feldspule 34 bzw. die zweite Feldspule 36 eingelegt sind. An seinen beiden stirnseitigen Enden ist der zweite Wickelkörper 28 in Anlage an den ersten und zweiten Einstellelementen 24, 26, mittels denen der zweite Wickelkörper 28 mit den von ihm getragenen Feldspulen 34, 36 in Bezug auf den ersten Wickelkörper 12 mit seinen Sensorspulen 20, 22 in Axialrichtung positioniert werden kann.

Im dargestellten ersten Ausführungsbeispiel ist der axiale Abstand der beiden Sensorspulen 20, 22 kleiner als der axiale Abstand der beiden Feldspulen 34, 36. Der Unterschied der axialen Abstände ist dabei geringer als die axiale Ausdehnung eines Wickelfensters 30, 32 für die Feldspulen 34, 36. Durch Verschiebung der axialen Position des zweiten Wickelkörpers 28 lassen sich Offsetsignale der Sensorspulen 20, 22 ausgleichen. Dies ist besonders gut möglich, wenn sich die Sensorspule 20, 22 in der Nähe der zugehörigen Feldspule 34, 36 befindet, insbesondere die axiale Erstreckung der Feldspule 34, 36 sich überlappt mit der axialen Erstreckung der Sensorspulen 20, 22.

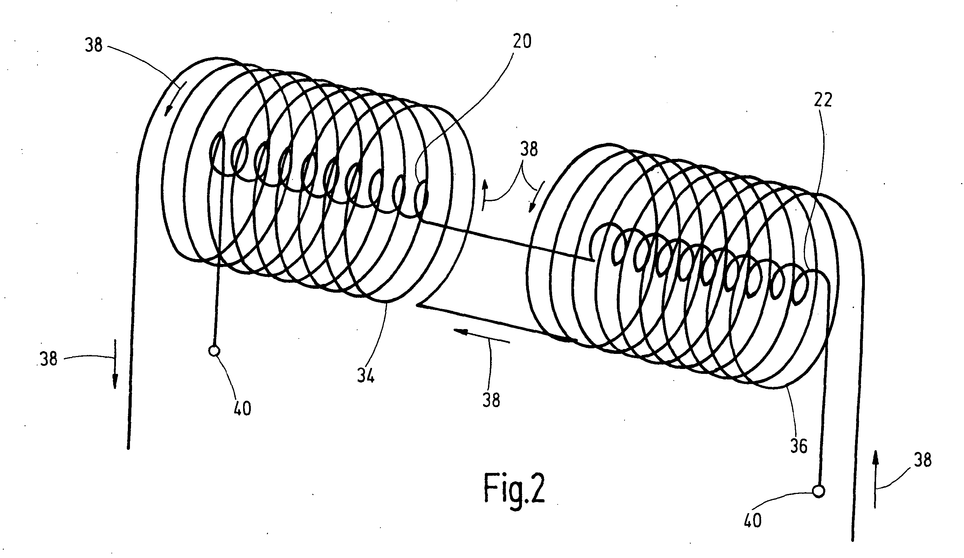

Die Fig. 2 zeigt schematisch die Anordnung der Sensorspulen 20, 22 in Bezug auf die Feldspulen 34, 36 der Fig. 1, wobei aus Gründen der Darstellbarkeit in der Fig. 2 der Durchmesser der Sensorspulen 20, 22 kleiner gewählt ist und gleichzeitig der Durchmesser der Feldspulen 34, 36 größer gewählt ist, als dies nach der Darstellung in der Fig. 1 der Fall ist. Die Feldspulen 34, 36 sind aus einem einzigen Stück Draht gewickelt und werden vom Feldstrom 38 erregt, dessen Fließrichtung durch die Feldspulen 34, 36 aus Gründen der Übersichtlichkeit an mehreren Stellen in der Fig. 2 angegeben ist. Infolge des Feldstromes 38, bei dem es sich um einen Wechselstrom geeigneter Frequenz handelt, beispielsweise 50 kHz bis 50 MHz, insbesondere etwa 100 kHz bis 10 MHz und vorzugsweise etwa 1 MHz, wird in den Sensorspulen 20, 22 eine Sensorspannung induziert, der aufgrund der gegensinnigen Wicklung der beiden Sensorspulen 20, 22, die ebenfalls aus einem einzigen Stück gewickelt sind, im Idealfall null betragen kann. Die Sensorspannung wird über die Anschlusselektroden 40 einer nicht dargestellten Auswerteeinrichtung zugeführt, die einen ausreichend hochohmigen Eingang aufweist.

Durchströmt der Partikel 2 den von der ersten Feldspule 34 abgedeckten Bereich, ändert sich die in der ersten Sensorspule 20 induzierte Spannung derart, dass an den Anschlusselektroden 40 ein Detektionssignal abgegriffen werden kann. Dieses wird sich über der Zeit in Abhängigkeit von der Position des Partikels 2 in Bezug auf die erste Feldspule 34 bzw. die erste Sensorspule 20 ändern. Kommt infolge der Fluidströmung der Partikel 2 anschließend in den Bereich der zweiten Feldspule 36 bzw. der zweiten Sensorspule 22, ändert sich wiederum das an den Anschlusselektroden 40 anstehende Signal. Die Signaländerung kann dabei von umgekehrter Polarität sein. In jedem Fall ist durch den Signalverlauf auch ein Rückschluss auf die Strömungsgeschwindigkeit des Partikels 2 und mithin auf die Strömungsgeschwindigkeit des Fluids möglich.

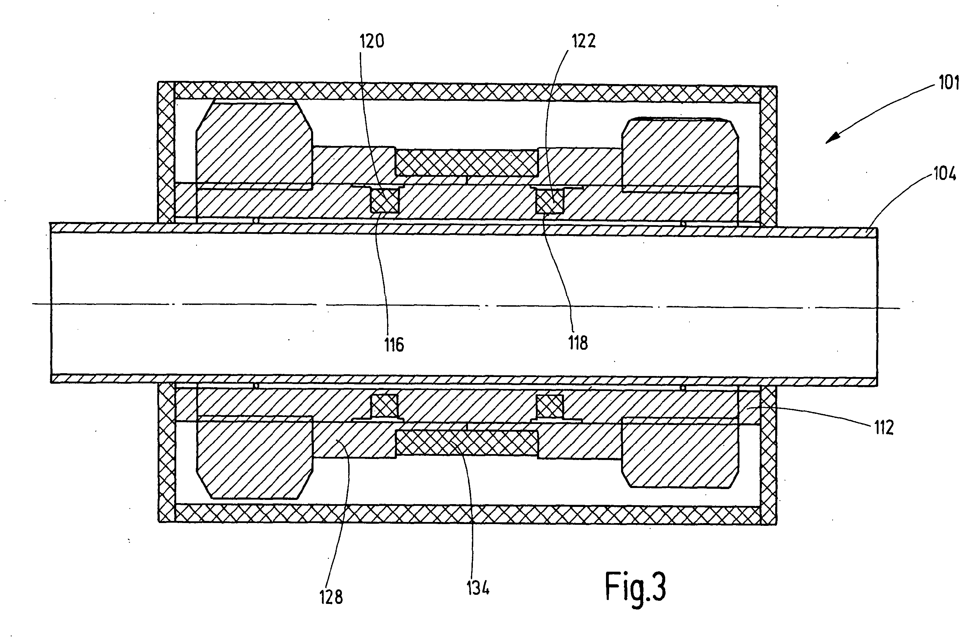

Die Fig. 3 zeigt einen Längsschnitt durch ein zweites Ausführungsbeispiel einer erfindungsgemäßen Vorrichtung 101. Der erste Wickelkörper 112 mit den beiden Sensorspulen 120, 122 ist übereinstimmend mit dem ersten Ausführungsbeispiel der Fig. 1 ausgeführt. Der zweite Wickelkörper 128 trägt eine einzige Feldspule 134, deren axiale Erstreckung größer ist als die lichte Weite zwischen den beiden Wickelfenstern 116, 118 des ersten Wickelkörpers 112. Gleichzeitig ist die axiale Erstreckung der Feldspule 134 aber kleiner als der Mittenabstand der beiden Sensorspulen 120, 122. Dadurch ist wiederum eine Überlappung von Feldspule 134 und Sensorspulen 120, 122 in Axialrichtung gegeben, wobei im zweiten Ausführungsbeispiel die beiden Sensorspulen 120, 122 axial über die Feldspule 134 hinausragen.

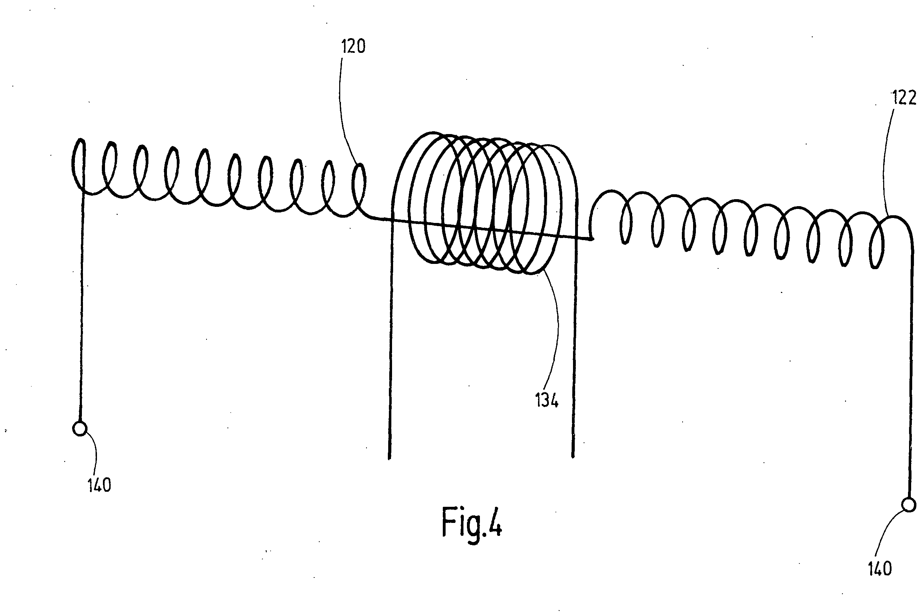

Die Fig. 4 zeigt schematisch die zu der Anordnung zu der Fig. 3 zugehörige Spulenanordnung, wobei in der Darstellung der Fig. 4 keine axiale Überlappung zwischen der Feldspule 134 und den Sensorspulen 120, 122 dargestellt ist. Die beiden Sensorspulen 120, 122 sind wiederum gegensinnig gewickelt und vorzugsweise aus einem einzigen Stück Draht hergestellt. An den Anschlusselektroden 140 kann das Sensorsignal abgegriffen und insbesondere einer Auswerteeinrichtung zugeführt werden.

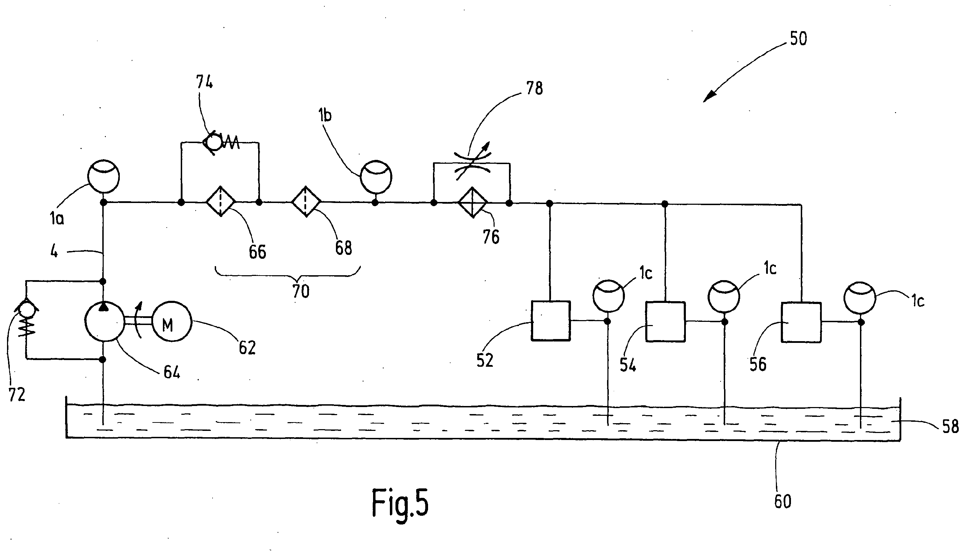

Die Fig. 5 zeigt ein erfindungsgemäßes System 50 zum Kühlen und/oder Schmieren von Komponenten 52, 54, 56 einer Antriebseinheit mittels eines Fluids 58, das in einem offenen oder geschlossenen Auffangraum 60 vorgehalten wird, beispielsweise in einem Getriebesumpf. Aus dem Auffangraum 60 wird das Fluid 58 mittels einer durch einen Motor 62 angetriebenen Pumpe 64 in die Leitung 4 gepumpt. Eine erste erfindungsgemäße Vorrichtung 1a ist in den Abschnitt zwischen der Pumpe 64 und der aus einem Feinfilter 66 und einem Grobfilter 68 bestehenden Filtereinheit 70 angeordnet. Parallel zur Pumpe 64 und zum Feinfilter 66 ist jeweils ein Bypass-Ventil 72, 74 geschaltet. Zwischen der Filtereinheit 70 und einem Wärmetauscher 76 mit Thermobypass 78 ist eine zweite erfindungsgemäße Vorrichtung 1b geschaltet. In der ersten und zweiten Vorrichtung 1a, 1b können beispielsweise Partikel mit einer Größe zwischen 50 und 200 *µ*m detektiert werden. Die erste Vorrichtung 1a kann in einem die Pumpe 64 mit der Filtereinheit 70 verbindenden Verbindungsflansch angeordnet sein und insbesondere integral mit diesem ausgebildet sein. Die erste Vorrichtung 1a ist vorgesehen für eine Gesamtsystemüberwachung. Die zweite Vorrichtung 1b ist vorgesehen für eine Überwachung der Systemreinheit.

Im Anschluss an den Wärmetauscher 76 wird das Fluid den einzelnen Komponenten 52, 54, 56 zugeführt, bei denen es sich beispielsweise um eine Schmierstelle für ein Lager, für ein Zahnradpaar oder für eine Buchse handeln kann. Im Leitungsabschnitt zwischen den Komponenten 52, 54, 56 und dem Auffangraum 60 kann jeweils eine weitere erfindungsgemäße Vorrichtung 1c angeordnet sein, die optimiert ist für eine Verschleißüberwachung der jeweiligen Komponente 52, 54, 56 und Partikel in der Größe zwischen 50 und 500 *µ*m detektieren kann.

Sofern möglich oder auch für eine Nachrüstung zugänglich, wäre die Position nach der Komponente 52, 54, 56 für die Anordnung der Vorrichtung 1c besonders günstig, weil die dort gemessene Partikelbelastung unmittelbar Rückschlüsse auf den Zustand der Komponente 52, 54, 56 zulässt. Vorteilhaft wäre auch ein Vergleich der gemessenen Partikelbelastungen an der Position nach der Filtereinheit 70 und an der Position nach der Komponente 52, 54, 56, weil dadurch der Partikeleintrag durch die Komponente 52, 54, 56 ermittelt werden könnte. Insbesondere wenn diese Positionen nicht zugänglich sind ist die Position vor oder nach der Pumpe 64 vorteilhaft, weil sich dort die Vorrichtung 1a besonders einfach nachrüsten lässt.

## Patentansprüche

1. Vorrichtung (1) zum Detektieren von Partikeln (2) in einem in einer Leitung (4) strömenden Fluidstrom (6), insbesondere induktiver Partikelzähler, wobei die Vorrichtung (1) mindestens eine Feldspule (34, 36) zum Erzeugen eines den Fluidstrom (6) mindestens abschnittsweise abdeckenden Magnetfeldes aufweist und eine Sensorspule (20, 22), die mit einer Auswerteeinrichtung verbindbar ist, mittels der aus dem in der Sensorspule (20, 22) induzierten Signal die Anwesenheit eines Partikels (2) in dem Fluidstrom (6) detektierbar ist, wobei die Vorrichtung (1) mindestens eine erste und eine zweite Sensorspule (20, 22) aufweist, und wobei die beiden Sensorspulen (20, 22) gegensinnig zueinander gewickelt und koaxial zu der Feldspule (34, 36) angeordnet sind, **dadurch gekennzeichnet, dass** der in Bezug auf die Strömungsrichtung und in Bezug auf die Mittelachse (8) der Leitung (4) radiale Abstand der Sensorspulen (20, 22) von dem Fluidstrom (6) von dem radialen Abstand der Feldspule (34, 36) von dem Fluidstrom (6) abweicht.

2. Vorrichtung (1) nach Anspruch 1, **dadurch gekennzeichnet, dass** die beiden Sensorspulen (20, 22) miteinander verbunden sind, insbesondere dass die beiden Sensorspulen (20, 22) in Reihe geschaltet sind.

3. Vorrichtung (1) nach Anspruch 1 oder 2, **dadurch gekennzeichnet, dass** die beiden Sensorspulen (20, 22) in Bezug auf eine Richtung des Fluidstroms (6) axial voneinander beabstandet sind.

4. Vorrichtung (1) nach einem der Ansprüche 1 bis 3, **dadurch gekennzeichnet, dass** die Sensorspulen (20, 22) auf einen ersten Wickelkörper (12) gewickelt sind, der einen Abschnitt einer Leitung (4) umgibt, innerhalb der das Fluid (58) strömt.

5. Vorrichtung (1) nach Anspruch 4, **dadurch gekennzeichnet, dass** der erste Wickelkörper (12) von der Leitung (4) mechanisch entkoppelt ist, insbesondere nur über einen elastisch verformbaren Abstandshalter (14) in Anlage an der Leitung (4) ist.

6. Vorrichtung (1) nach einem der Ansprüche 1 bis 5, **dadurch gekennzeichnet, dass** die Feldspule (34, 36) mittels einer Einstelleinrichtung gegenüber den Sensorspulen (20, 22) in Bezug auf eine Strömungsrichtung des Fluids (58) axial verschiebbar ist.

7. Vorrichtung nach Anspruch 6, **dadurch gekennzeichnet, dass** die Feldspule (34, 36) auf einen zweiten Wickelkörper (28) gewickelt ist, der mittels der Einstelleinrichtung gegenüber einem die Sensorspulen (20, 22) tragenden ersten Wickelkörper (12) verschiebbar ist.

8. Vorrichtung (1) nach einem der Ansprüche 1 bis 7, **dadurch gekennzeichnet, dass** die Vorrichtung (1) zwei gleichsinnig gewickelte Feldspulen (20, 22) aufweist, die miteinander verbunden sind, insbesondere in Reihe geschaltet sind.

9. System (50) zum Kühlen und/oder Schmieren von Komponenten (52, 54, 56) einer Antriebseinheit mittels eines Fluids (58), insbesondere zum Kühlen und/oder Schmieren eines Getriebes einer Windkraftanlage, mit einer Pumpe (64) und einem Filter (66, 68), die in einem Kühl- und/oder Schmiermittelkreislauf angeordnet sind, **dadurch gekennzeichnet, dass** das System (50) eine Vorrichtung (1) nach einem der Ansprüche 1 bis 8 aufweist, und dass die Vorrichtung (1) in dem Kühl- und/oder Schmiermittelkreislauf angeordnet ist.

10. System (50) nach Anspruch 9, **dadurch gekennzeichnet, dass** die Vorrichtung (1, 1a) zwischen der Pumpe (64) und dem Filter (66, 68) angeordnet ist, insbesondere an einem Verbindungselement von Pumpe (64) und Filter (66, 68).

11. System (50) nach Anspruch 9 oder 10, **dadurch gekennzeichnet, dass** die Vorrichtung (1, 1b) zwischen dem Filter (66, 68) und einer zu kühlenden und/oder schmierenden Komponente (52, 54, 56) angeordnet ist.

12. System (50) nach einem der Ansprüche 9 bis 11, **dadurch gekennzeichnet, dass** die Vorrichtung (1, 1c) zwischen einer zu kühlenden und/oder schmierenden Komponente (52, 54, 56) und einem Rücklaufpfad zu einem Auffangraum (60) für das Fluid (58) angeordnet ist.

## Claims

1. An apparatus (1) for detecting particles (2) in a fluid flow (6) flowing in a pipeline (4), in particular an inductive particle counter, the apparatus (1) having at least one field coil (34, 36) for generating a magnetic field which covers at least sections of the fluid flow (6), and a sensor coil (20, 22) which can be connected to an evaluation device by means of which the presence of a particle (2) in the fluid flow (6) can be detected from the signal induced in the sensor coil (20, 22), the apparatus (1) having at least a first and a second sensor coil (20, 22), and the two sensor coils (20, 22) being wound in opposite directions and being arranged coaxially to the field coil (34, 36), **characterised in that** the radial distance between the sensor coils (20, 22) and the fluid flow (6) relative to the flow direction and relative to the centre axis (8) of the pipe line (4) differs from the radial distance between the field coil (34, 36) and the fluid flow (6).

2. The apparatus (1) according to Claim 1, **characterised in that** the two sensor coils (20, 22) are connected to one another, in particular that the two sensor coils (20, 22) are connected in series.

3. The apparatus (1) according to Claim 1 or 2, **characterised in that** the two sensor coils (20, 22) are spaced apart from one another axially relative to a direction of the fluid flow (6).

4. The apparatus (1) according to any of Claims 1 to 3, **characterised in that** the sensor coils (20, 22) are wound onto a first winding body (12) which surrounds a section of a pipeline (4) within which the fluid (58) flows.

5. The apparatus (1) according to Claim 4, **characterised in that** the first winding body (12) is decoupled mechanically from the pipeline (4), in particular is only in contact with the pipeline (4) via an elastically deformable spacer (14).

6. The apparatus (1) according to any of Claims 1 to 5, **characterised in that** the field coil (34, 36) can be displaced axially with respect to the sensor coils (20, 22) relative to a flow direction of the fluid (58) by means of an adjusting device.

7. The apparatus (1) according to Claim 6, **characterised in that** the field coil (34, 36) is wound onto a second winding body (28) which can be displaced with respect to a first winding body (12) supporting the sensor coils (20, 22) by means of the adjusting device.

8. The apparatus (1) according to any of Claims 1 to 7, **characterised in that** the apparatus (1) has two field coils (20, 22) wound in the same direction and which are connected to one another, in particular are connected in series.

9. A system (50) for cooling and/or lubricating components (52, 54, 56) of a drive unit by means of a fluid (58), in particular for cooling and/or lubricating a gearing mechanism of a wind turbine, having a pump (64) and a filter (66, 68) which are disposed within a cooling and/or lubricant circuit, **characterised in that** the system (50) has an apparatus (1) according to any of Claims 1 to 8, and that the apparatus (1) is disposed within the cooling and/or lubricant circuit.

10. The system (50) according to Claim 9, **characterised in that** the apparatus (1, 1a) is disposed between the pump (64) and the filter (66, 68), in particular on a connection element of the pump (64) and the filter (66, 68).

11. The system (50) according to Claim 9 or 10, **characterised in that** the apparatus (1, 1b) is disposed between the filter (66, 68) and a component (52, 54, 56) to be cooled and/or lubricated.

12. The system (50) according to any of Claims 9 to 11, **characterised in that** the apparatus (1, 1c) is disposed between a component (52, 54, 56) to be cooled and/or lubricated and a return flow path to a collecting space (60) for the fluid (58).

## Revendications

1. Système (1) de détection de particules (2) dans un courant (6) de fluide passant dans un conduit (4), notamment compteur de particules inductif, le système (1) ayant au moins une bobine (34, 36) de champ pour produire un champ magnétique recouvrant, au moins par endroit, le courant (6) de fluide, et une bobine (20, 22) de capteur, qui peut être reliée à un dispositif d'exploitation au moyen duquel on peut, à partir du signal induit dans la bobine (20, 22) de capteur, détecter la présence d'une particule (2) dans le courant (6) de fluide, le système (1) ayant au moins une première et une deuxième bobines (20, 22) de capteur et dans lequel les deux bobines (20, 22) de capteur sont bobinées en sens contraire l'une à l'autre et sont disposées coaxialement à la bobine (34, 36) de champ, **caractérisé en ce que** la distance radiale, rapportée à la direction d'écoulement et rapportée à l'axe (8) médian du conduit (4), des bobines (20, 22) de capteur au courant (6) de fluide diffère de la distance radiale des bobines (34, 36) de champ au courant (6) de fluide.

2. Système (1) suivant la revendication 1, **caractérisé en ce que** les deux bobines (20, 22) de capteur sont reliées entre elles, notamment **en ce que** les deux bobines (20, 22) de capteur sont montées en série.

3. Système (1) suivant la revendication 1 ou 2, **caractérisé en ce que** les deux bobines (20, 22) de capteur sont, rapporté à une direction du courant (6) de fluide, à distance axialement l'une de l'autre.

4. Système (1) suivant l'une des revendications 1 à 3, **caractérisé en ce que** les bobines (20, 22) de capteur sont bobinées sur un premier corps (12) de bobine, qui entoure un tronçon d'un conduit (4), dans lequel passe le fluide (58).

5. Système (1) suivant la revendication 4, **caractérisé en ce que** le premier corps (12) de bobine est découplé mécaniquement du conduit (4), en étant en contact avec le conduit (4) seulement par l'intermédiaire d'une entretoise (14) déformable élastiquement.

6. Système (1) suivant l'une des revendications 1 à 5, **caractérisé en ce que** la bobine (34, 36) de champ peut, au moyen d'un dispositif de réglage, être déplacée axialement, rapporté à une direction d'écoulement du fluide (58) par rapport aux bobines (20, 22) de capteur.

7. Système (1) suivant la revendication 6, **caractérisé en ce que** la bobine (34, 36) de champ est bobinée sur un deuxième corps (28) de bobine, qui peut, au moyen du dispositif de réglage, être déplacé par rapport à un premier corps (12) de bobine portant les bobines (20, 22) de capteur.

8. Système (1) suivant l'une des revendications 1 à 7, **caractérisé en ce que** le système a deux bobines (20, 22) de champ bobinées dans le même sens, qui sont reliées entre elles, en étant notamment montées en série.

9. Ensemble (50) de refroidissement et/ou de lubrification d'éléments (52, 54, 56) d'un groupe d'entraînement, au moyen d'un fluide (58), notamment pour refroidir et/ou lubrifier une transmission d'une éolienne, comprenant une pompe (64) et un filtre (66, 68), qui sont montés dans un circuit de fluide de refroidissement et/ou de lubrification, **caractérisé en ce que** l'ensemble (50) a un système (1) suivant l'une des revendications 1 à 8 et **en ce que** le système (1) est monté dans le circuit de fluide de refroidissement et/ou de lubrification.

10. Ensemble (50) suivant la revendication 9, **caractérisé en ce que** le système (1, 1a) est monté entre la pompe (64) et le filtre (66, 68), notamment sur un élément de liaison de la pompe (64) et du filtre (66, 68).

11. Ensemble (50) suivant la revendication 9 ou 10, **caractérisé en ce que** le système (1, 1b) est monté entre le filtre (66, 68) et un élément (52, 54, 56) à refroidir et/ou à lubrifier.

12. Ensemble (50) suivant l'une des revendications 9 à 11, **caractérisé en ce que** le système (1, 1c) est monté entre un élément (52, 54, 56) à refroidir et/ou à lubrifier et un trajet de retour à un espace (60) de collecte du fluide (58).
